(19)

Europäisches
Patentamt
European
Patent Office
Office européen
des brevets

(11) **EP 2 598 167 B1**

(12) **EUROPEAN PATENT SPECIFICATION**

(45) Date of publication and mention
of the grant of the patent:
**01.04.2015 Bulletin 2015/14**

(21) Application number: **11738789.4**

(22) Date of filing: **29.07.2011**

(51) Int Cl.:
*A61K 39/00* (2006.01)      *A61K 39/395* (2006.01)
*A61K 47/34* (2006.01)      *C07K 16/00* (2006.01)
*C07K 16/22* (2006.01)      *C07K 16/28* (2006.01)
*C07K 16/32* (2006.01)

(86) International application number:
**PCT/GB2011/051440**

(87) International publication number:
**WO 2012/013980 (02.02.2012 Gazette 2012/05)**

(54) **STABILIZED AQUEOUS ANTIBODY COMPOSITIONS**

STABILISIERTE WÄSSRIGE ANTIKÖRPERZUSAMMENSETZUNGEN

COMPOSITIONS AQUEUSES STABILISÉES D'ANTICORPS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.07.2010 US 369161 P**

(43) Date of publication of application:
**05.06.2013 Bulletin 2013/23**

(73) Proprietor: **Arecor Limited
Cambridge CB4 0FE (GB)**

(72) Inventors:
• **JEZEK, Jan
Cambridgeshire CB4 0FE (GB)**
• **DERHAM, Barry Kingston
Cambridgeshire CB4 0FE (GB)**
• **ZAPADKA, Karolina
Cambridgeshire CB4 0FE (GB)**

(74) Representative: **Teuten, Andrew John
Sagittarius IP
Three Globeside
Fieldhouse Lane
Marlow, Buckinghamshire SL7 1HZ (GB)**

(56) References cited:
**WO-A1-2010/035001**

• **BRYJAK J ET AL: "Storage stabilization and
purification of enzyme by water-soluble synthetic
polymers", ENZYME AND MICROBIAL
TECHNOLOGY, STONEHAM, MA, US, vol. 16, no.
7, 1 July 1994 (1994-07-01), pages 616-621,
XP023679672, ISSN: 0141-0229, DOI:
10.1016/0141-0229(94)90128-7 [retrieved on
1994-07-01]**
• **ANDERSSON M M ET AL: "Protein stabilising
effect of polyethyleneimine", JOURNAL OF
BIOTECHNOLOGY, ELSEVIER SCIENCE
PUBLISHERS, AMSTERDAM, NL, vol. 72, no. 1-2,
11 June 1999 (1999-06-11), pages 21-31,
XP004172884, ISSN: 0168-1656, DOI:
10.1016/S0168-1656(99)00050-4**

EP 2 598 167 B1

**Description**

BACKGROUND OF THE INVENTION

Although a variety of chemical processes, such as oxidation, deamidation and aspartate isomerisation, may affect critical quality attributes of therapeutic proteins, such as antibodies, protein aggregation is arguably the most common process affecting protein stability. Aggregation is typically exacerbated and is the key degradation pathway of proteins formulated in aqueous solution at high concentrations, such as 10 mg/ml or greater. During storage, aggregation can lead to an unacceptably high level of high molecular weight species (HMWS) in the formulation or to formation of larger insoluble aggregates (particulates). Such contaminated formulations may fall outside the specification set by the U.S. Food and Drug Administration and other pharmaceutical regulatory authorities.

To some extent, protein aggregation can be controlled by optimization of various parameters of the protein composition. For example, methods to control the rate of aggregation may involve optimization of pH, addition of a metal ion chelator or addition of a surfactant.

The ionic strength of the composition can also affect the rate of aggregation in aqueous protein compositions. Conventional formulation development for a therapeutic protein therefore typically includes screening of tonicity modifiers, which can be selected from uncharged chemical species, such as sugars, or a charged chemical species, such as an inorganic or an organic salt. An uncharged tonicity modifier is typically preferred if the rate of aggregation is lower in low ionic strength compositions, while a charged tonicity modifier is preferred if the rate of aggregation is lower in higher ionic strength compositions. The charged tonicity modifiers typically used in aqueous protein compositions for therapeutic applications include sodium chloride. Typical uncharged tonicity modifiers include sucrose, trehalose, glycerol and mannitol.

Although high ionic strength may be beneficial for controlling aggregation, this may be accompanied by unacceptably high osmolarity, causing pain to the patient when the formulation is injected, particularly in the case of subcutaneous or intramuscular injection. Ideally, a composition for injection, particularly subcutaneous or intramuscular injection, should be approximately isotonic, i.e. have an osmolarity between about 280 and about 340 mOsm/L.

Protein aggregation is a very complex process, involving a number of different mechanisms. However, it is believed that two dominant types of non-covalent interactions drive the protein aggregation: (1) hydrophobic interactions between non-polar parts of the protein molecules, and (2) charge-charge interactions between charged regions of the protein molecules. It is believed that in those cases where the rate of aggregation is lower in compositions of higher ionic strength than in compositions of lower ionic strength the key cause of aggregation is due to charge-charge interactions between the protein molecules.

There is a need for improved methods for preparing stable, highly concentrated protein solutions, particularly highly concentrated antibody solutions.

WO2010/035001 (Stabilitech Ltd) discloses that polypeptide preparations mixed with an aqueous solution containing one, two or more sugars and a polyethyleneimine (PEI) are preserved well on drying such as freeze drying. Bryjak, J. et al. (Enzyme Microb. Technol., 1994, 16, 616-621) discloses penicillin acylase purification and storage stabilization in the presence of water-soluble polymers. Andersson, M. M. et al. (J. Biotech., 1999, 72, 21-31) discloses investigations on the effect of PEI on the protein stability towards storage and other stresses of freezing and drying. Most of the investigations were performed with porcine muscle lactate dehydrogenase as a model.

SUMMARY OF THE INVENTION

The present invention addresses the problem of aggregation of antibody proteins, in particular, antibody proteins at elevated concentrations. Application of the present invention results in considerable reduction of the rate of aggregation in aqueous antibody protein compositions. The present invention also addresses the problem of self-association of antibody proteins and in aqueous compositions of antibody proteins, particularly at high antibody protein concentrations.

In one embodiment, the invention relates to an aqueous solution for administration to a subject by subcutaneous, intravenous or intramuscular injection comprising an antibody protein at a concentration of at least about 10 mg/mL and a stabilizing amount of polyethyleneimine, wherein the weight ratio of antibody protein to polyethyleneimine is at least 20.

In one embodiment, the invention provides a method of reducing the rate of aggregation of an antibody protein in an aqueous solution for administration to a subject by subcutaneous, intravenous or intramuscular injection. The method comprises the step of adding to the solution a stabilizing amount of polyethyleneimine wherein the weight ratio of antibody protein to polyethyleneimine is at least 20, and wherein the concentration of the antibody protein in the solution is at least about 10 mg/mL.

Disclosed herein is a method of reducing the rate of viscosity increase during storage, of an aqueous antibody protein solution. The method comprises the step of adding to the solution an effective amount of polyethyleneimine. The concentration of the antibody protein in the solution is at least about 10 mg/mL.

Also disclosed is a method of reducing the rate of undesired fragmentation of antibody proteins, as detected by the formation of low molecular weight species during storage. In particular, such undesired fragmentation may occur in fusion proteins comprising one or more antibody fragments. The method comprises the step of adding to the solution an effective amount of polyethyleneimine. The concentration of the antibody protein in the solution is at least about 10 mg/mL.

Also disclosed is a method for administering a therapeutic antibody protein to a subject in need thereof. The method comprises the step of administering an aqueous solution comprising the antibody protein at a concentration of at least about 10 mg/mL, and a stabilizing amount of polyethyleneimine. The composition is administered by intravenous, subcutaneous or intramuscular injection.

DETAILED DESCRIPTION OF THE INVENTION

The present invention relates to the discovery that polyethyleneimine can stabilize highly concentrated aqueous antibody protein solutions.

The present invention is applicable to aqueous compositions of antibody proteins for therapeutic applications.

Compared with existing methods for stabilizing high concentration aqueous formulations of antibody proteins, particularly with respect to reduced rate of aggregation and reversible self-association, this invention offers several advantages. For example, the present invention allows a more rational approach to formulation development, requiring less trial and error in designing trial formulations. In turn, this enables an accelerated, lower cost route to an optimized formulation meeting the key performance requirements of storage stability and suitability for low volume subcutaneous injection.

Compared with prior art methods, the stability benefits conferred by the present invention enable the use of higher concentration aqueous formulations of therapeutically important antibody proteins.

In one embodiment, the invention provides an aqueous solution for administration to a subject by subcutaneous. intravenous or intramuscular injection comprising an antibody protein at a concentration of at least about 10 mg/mL and a stabilizing amount of a polyethyleneimine, wherein the weight ratio of antibody protein to polyethyleneimine is at least 20. The antibody protein is preferably a therapeutic antibody protein. Such an antibody protein has a desirable therapeutic or prophylactic activity and is indicated for the treatment, inhibition or prevention of a disease or medical disorder.

The term "antibody protein", as used herein, refers to an antibody, an antibody fragment, an antibody conjugated to an active moiety, a fusion protein comprising one or more antibody fragments, such as an immunoglobulin Fc domain, or a derivative of any of the aforementioned. Preferred antibodies include monoclonal antibodies and polyclonal antibodies, preferably monoclonal antibodies. The monoclonal antibodies can be, for example, mammalian or avian, chimeric, for example, human/mouse or human/primate chimeras, humanized antibodies or fully human antibodies. Suitable antibodies include an immunoglobulin, such as IgG, including $IgG_1$, $IgG_2$, $IgG_3$ or $IgG_4$, IgM, IgA, such as $IgA_1$ or $IgA_2$, IgD, IgE or IgY. Suitable antibodies also include single chain antibodies. Also included are antibody fragments including Fc, Fab, $Fab_2$, ScFv fragments and the like. Also embraced are single domain antibodies including Nanobodies.

The term "aqueous solution", as used herein, refers to a solution in water, preferably distilled water, deionized water, water for injection, sterile water for injection or bacteriostatic water for injection. The aqueous solutions of the invention include dissolved antibody protein, polyethyleneimine and, optionally, one or more additives and/or excipients. The aqueous solutions can also include one or more components, such as additives or excipients, which are partially dissolved or undissolved. The presence of such component or components will result in a multi-phase composition, such as a suspension or an emulsion. Preferably, the aqueous solution of the invention is a homogeneous solution, as determined by eye or by light-scattering.

The term "stabilizing amount of polyethyleneimine" refers to a polyethyleneimine concentration that is sufficient to significantly reduce the rate of antibody protein aggregation in a composition, such as an aqueous antibody protein solution, compared with a composition lacking polyethyleneimine but otherwise identical, following storage under the same conditions and for the same length of time.

In one embodiment, the polyethyleneimine is present in the composition at a concentration which is sufficient to limit the increase in high molecular weight protein species to no more than 5% (by weight of total protein) after storage at 40°C for one month. In one embodiment, polyethyleneimine is present at a concentration that is sufficient to limit the increase in high molecular weight protein species to no more than 5% (by weight of total protein) after storage at 2-8°C for up to two years. Quantitation of high molecular weight species is as percent by weight of the total protein in the composition.

In one embodiment, the polyethyleneimine is present in the composition at a concentration which is sufficient to limit the increase in high molecular weight protein species by at least 10%, preferably by at least 25%, compared with a composition lacking the polyethyleneimine but otherwise identical, following storage under the same conditions and length of time.

In one embodiment, the polyethyleneimine is present in the composition at a concentration which is sufficient to maintain an aqueous composition of a protein free of visible aggregates while formation of visible aggregates is observed in a composition lacking polyethyleneimine but otherwise identical, following storage under the same conditions and for the same length of time. Quantification of visible aggregates can be performed by turbidity or other types of light scattering measurement.

In certain embodiments, the antibody is fused or conjugated to an active molecule, such as a toxin or a chelating agent capable of binding a radioactive metal ion, such as $^{99}$Tc, $^{111}$Ir, $^{131}$I or $^{90}$Y. In such embodiments, the antibody typically functions as a targeting agent, for example, directing the active molecule to cells which display a certain cell surface protein.

Specific antibodies which can be formulated as described herein include, but are not limited to, infliximab (chimeric antibody, anti-TNF$\alpha$), adalimumab (human antibody, anti-TNF$\alpha$), basiliximab (chimeric antibody, anti-IL-2), abciximab (chimeric antibody, anti-GpIIb/IIIa), daclizumab (humanized antibody, anti-IL-2), gemtuzumab (humanized antibody, anti-CD33), alemtuzumab (humanized antibody, anti-CD52), edrecolomab (murine Ig2a, anti-EpCAM), rituximab (chimeric antibody, anti-CD20), palivizumab (humanized antibody, anti-respiratory syncytial virus), trastuzumab (humanized antibody, anti- HER2/neu(erbB2) receptor), bevacizumab (humanized antibody, anti-VEGF), cetuximab (chimeric antibody, anti-EGFR), eculizumab (humanized antibody, anti- complement system protein C5), efalizumab (humanized antibody, anti-CD 1Ia), ibritumomab (murine antibody, anti-CD20), muromonab-CD3 (murine antibody, anti-T cell CD3 receptor), natalizumab (humanized antibody, anti-$\alpha$4 integrin), nimotuzumab (humanized IgG1, anti-EGF receptor), omalizumab (humanized antibody, anti-IgE), panitumumab (human antibody, anti-EGFR), ranibizumab (humanized antibody, anti-VEGF), 1-131 tositumomab (humanized antibody, anti-CD20), ofatumumab (human antibody, anti-CD-20), certolizumab (humanized antibody, anti-TNF-$\alpha$), golimumab (human antibody, anti-TNF$\alpha$) and denosumab (human antibody, anti-RANK ligand). Preferred antibodies include trastuzumab and rituximab.

Other chimeric antibodies which can be formulated as described herein include bavituximab (anti-phosphatidylserine), brentuximab (anti-CD30), siltuximab (anti-IL-6), clenoliximab (anti-CD4), galiximab (anti-CD80), gomiliximab (anti-CD23), keliximab (anti-CD4), lumiliximab (anti-CD23), priliximab (anti-CD4), teneliximab (anti-CD40), vapaliximab (anti-VAP1), ecromeximab (anti-GD3), and pagibaximab (anti-staphylococcal lipoteichoic acid).

Other humanized antibodies which can formulated as described herein include epratuzumab (anti-CD22), afutuzumab (anti-CD20), bivatuzumab mertansine (anti-CD44), cantuzumab mertansine (anti-mucin), citatuzumab bogatox (anti-TACSTD1), dacetuzumab (anti-CD40), elotuzumab (anti-CD319), etaracizumab (anti-$\alpha_v\beta_3$-integrin), farletuzumab (anti-FR$\alpha$), inotuzumab ozogamicin (anti-CD22), labetuzumab (anti-carcinoembryonic antigen), lintuzumab (anti-CD33), milatuzumab (anti-CD74), nimotuzumab (anti-EGFR), oportuzumab monatox (anti-EpCAM), pertuzumab (anti-HER2), sibrotuzumab (anti-FAP), tacatuzumab tetraxetan (anti-alpha-fetoprotein), tigatuzumab (anti-TRAIL-2), tucotuzumab celmoleukin (anti-EpCAM), veltuzumab (anti-CD20), aselizumab (anti-CD62L), apolizumab (anti-HLA-DRB), benralizumab (anti-CD125), cedelizumab (anti-CD4), epratuzumab (anti-CD22), erlizumab (anti-CD18), fontolizumab (anti-interferon-$\gamma$), mepolizumab (anti-IL5), ocrelizumab (anti-CD20), pascolizumab (anti-IL4), pexelizumab (anti-complement component 5), PRO-140 (anti-CCR5), reslizumab (anti-IL5), rontalizumab (anti interferon-$\alpha$), rovelizumab (anti-CD11, CD18), siplizumab (anti-CD2), talizumab (anti-IgE), teplizumab (anti-CD3), tocilizumab (anti-IL6R), vedolizumab (anti-$\alpha_4\beta_7$-integrin), visilizumab (anti-CD3), ibalizumab (anti-CD4), tefibazumab (anti-clumping factor A), tadocizumab (anti-$\alpha_{11b}\beta_3$-integrin), bapineuzumab (anti-amyloid-$\beta$), solanezumab (anti-amyloid-$\beta$), tanezumab (anti-NGF), urtoxazumab (anti-E. coli Shiga-like toxin II B subunit), felvizumab (anti-respiratory syncytial virus), motavizumab (anti-respiratory syncytial virus glycoprotein F) and lebrikizumab (anti-IL13).

Additional human antibodies which can be formulated as described herein include atorolimumab (anti-Rh factor), fresolimumab (anti-TGF$\beta$-1, -2, and -3), lerdelimumab (anti-TGF$\beta$-2), metelimumab (anti-TGF$\beta$-1), morolimumab (anti-Rh factor), ipilimumab (anti-CTLA-4), tremelimumab (anti-CTLA-4), bertilimumab (anti-CCL11), zanolimumab (anti-CD4), briakinumab (anti-IL12, -23), canakinumab (anti-IL1$\beta$), ustekinumab (anti-IL12, -23), adecatumumab (anti-EpCAM), belimumab (anti-B cell activating factor), cixutumumab anti-IGF-1 receptor), conatumumab (anti-TRAIL-R2), figitumumab (anti-IGF-1 receptor), iratumumab (anti-CD30), lexatumumab (anti-TRAIL-R2), lucatumumab (anti-CD40), mapatumumab (anti-TRAIL-R4), necitumumab (anti-EGFR), olaratumab (anti-PDGF-R$\alpha$), pritumumab (anti-vimentin), robatumumab (anti-IGF-1 receptor), votumumab (anti-tumor antigen CTAA16.88), zalutumumab (anti-EGFR), stamulumab (anti-myostatin), efungumab (anti-fungal HSP90), exbivirumab (anti-hepatitis B surface antigen), foravirumab (anti- rabies glycoprotein), libivirumab (anti-hepatitis B surface antigen), rafivirumab (anti- rabies glycoprotein), regavirumab (anti-cytomegalovirus glycoprotein B), sevirumab (anti-cytomegalovirus), tuvirumab (anti-hepatitis B virus), panobacumab (anti-pseudomonas aeruginosa serotype IATS 011), raxibacumab (anti-anthrax toxin), ramucirumab (anti-VEGF-R2), and gantenerumab (anti-amyloid-$\beta$).

Fusion proteins comprising a fragment of an immunoglobulin molecule can also be formulated according to the invention. Suitable fusion proteins include proteins comprising an active protein domain fused to one or more immunoglobulin fragments, such as Fc domains. Such fusion proteins include dimeric proteins having monomeric units comprising an active protein domain, such as a soluble receptor or a receptor extracellular ligand binding domain, which is fused to an immunoglobulin Fc domain. Two Fc domains can associate via disulfide bonds to form the dimeric protein.

Such fusion proteins include etanercept, abatacept and belatacept.

The antibody protein can be isolated from natural sources or be a recombinant protein.

In certain embodiments, the antibody protein is substantially pure, that is, the composition comprises a single protein and no substantial amount of any additional protein. In preferred embodiments, the protein comprises at least 99%, preferably at least 99.5% and more preferably at least about 99.9% of the total protein content of the composition. In preferred embodiments the protein is sufficiently pure for use as in a pharmaceutical composition.

The concentration of the antibody protein in the aqueous solution is at least about 10 mg/mL, and is preferably in the range of about 25 mg/mL to about 400 mg/mL. In certain embodiments the concentration is at least about 25 mg/mL. In certain embodiments, the protein concentration is at least about 30 mg/mL, 40 mg/mL, 50 mg/mL, 60 mg/mL, 70 mg/mL, 80 mg/mL, 90 mg/mL or 100 mg/mL. More preferably the protein concentration is greater than 50 mg/mL e.g. at least about 80 mg/mL. The concentration can be up to about 400 mg/mL, for example up to about 350 mg/mL, 300 mg/mL, 250 mg/mL, 200 mg/mL or 175 mg/mL. Every concentration range bounded by one of the foregoing lower limits and one of the foregoing upper limits is contemplated herein.

Polyethyleneimine is available in a wide range of molecular weights. In certain cases, polyethyleneimine is end-capped, for example, with ethylenediamine. The polyethyleneimine can comprise linear polymer molecules, branched polymer molecules, or a combination of linear and branched polymer molecules. Within the scope of the invention, the polyethyleneimine can also be modified by conjugation to other functionalized chemical species, for example oligomeric and polymeric species such as histidine-lysine short peptides. Such modified forms of polyethyleneimine are described in M. Hashemi et al., Cancer Gene Therapy (2011) 18, 12-19.

The term "pharmaceutically acceptable", as used herein, refers to components of a pharmaceutical composition which are suitable for the intended use and mode of administration to the body of a human or an animal, such as a mammal, without undue adverse consequences, such as toxicity, irritation, and allergic response and with a reasonable risk/benefit ratio.

The polyethyleneimine can be added to the composition of the invention in the free base form. In this embodiment, the pH of the composition is preferably sufficiently low to protonate at least a portion of the basic groups of the polyethyleneimine. The polyethyleneimine can also be added to the composition as a salt of a suitable acid, such as a pharmaceutically acceptable acid. Suitable acids include hydrochloric acid, hydrobromic acid, citric acid, lactic acid, tartaric acid, phosphoric acid, methanesulfonic acid, acetic acid, formic acid, maleic acid, fumaric acid, malic acid, succinic acid, malonic acid, sulfuric acid, L-glutamic acid, tartaric acid, L-aspartic acid, pyruvic acid, mucic acid, benzoic acid, glucoronic acid, oxalic acid, and ascorbic acid. In one embodiment, the acid is a polyacid comprising two or more acidic groups.

The polyethyleneimine can have a range of molecular weights and/or molecular weight distributions. Preferably the polyethyleneimine has a number-average molecular weight of about 5000 Da or less, preferably about 2500 Da or less. In certain embodiments, the polyethyleneimine has a number average molecular weight in the range of about 600 to about 1800 Da. For example, the polyethyleneimine can have a number-average molecular weight (excluding any contribution from anionic counterions) of about 600, 1200 or 1800 Da. As used herein, the molecular weight of a particular polyethyleneimine refers to the free base equivalent, i.e. it does not include the counter anions.

The polyethyleneimine is present in the composition at a concentration which is sufficient to provide the desired stability. In one embodiment, the concentration of the polyethyleneimine is from about 0.5 to about 20 mg/mL, for example from about 1 to about 10 mg/mL. In a more preferred embodiment, the concentration of the polyethyleneimine is about 5 mg/mL or less, for example, from about 1 mg/mL to about 5 mg/mL, about 0.5 mg/mL to about 5 mg/mL, about 0.5 mg/mL to about 2.5 mg/mL or about 1 mg/mL to about 2.5 mg/mL. In certain embodiments, the concentration of polyethyleneimine is about 1 mg/mL. As used herein, the mass of polyethyleneimine in a composition of the invention refers to the free base equivalent, i.e. it does not include any counter anions, if present.

The ratio (wt/wt) of antibody protein to polyethyleneimine is at least 20. In certain embodiments the weight ratio of protein to polyethyleneimine is from about 20 to about 300, preferably from about 50 to about 200. In certain embodiments, the weight ratio of protein to polyethyleneimine is about 100.

The solutions of the invention preferably comprise a buffer. Typically the buffer is selected to provide a pH that will allow dissolution of the protein to the desired concentration. Preferably, the pH is sufficiently low that at least a portion of the basic groups in the polyethyleneimine are protonated. The buffer can also be selected to enhance protein stability.

In one embodiment, the composition of the invention is further stabilized as disclosed in WO2008084237 which describes a composition comprising a protein and one or more additives, characterized in that the system is substantially free of a conventional buffer, i.e. a compound with a $pK_a$ within 1 unit of the pH of the composition at the intended temperature range of storage of the composition. In this embodiment, the pH of the composition is set to a value at which the composition has maximum measurable stability with respect to pH; the one or more additives (displaced buffers) are capable of exchanging protons with the protein and have $pK_a$ values at least 1 unit more or less than the pH of the composition at the intended temperature range of storage of the composition. By keeping the protein at a suitable pH, at or near a value at which the measurable stability is maximal, in the absence of a conventional buffer, the storage stability of the protein can be increased substantially. In certain embodiments, storage stability can generally be enhanced further, possibly substantially, by use of additives having $pK_a$ between 1 to 5 pH units, preferably between 1 to 3 pH units, most preferably from 1.5 to 2.5 pH units, of the pH of the aqueous composition at the intended temperature range of storage of the composition.

The solutions of the invention can further include one or more conventional excipients, such as an inorganic salt, preferably a salt which is a combination of sodium, potassium, calcium, or ammonium, with chloride, sulfate, carbonate, sulfite, nitrate, lactate, succinate, acetate, maleate or lactate; an amino acid, preferably histidine, glycine, arginine or methionine; a sugar or sugar alcohol, preferably trehalose, sucrose, mannitol, raffinose, sorbitol, lactitol, glycerol, or 1,2-propanediol; a surfactant, preferably polysorbate 20, polysorbate 60, polysorbate 80, poloxamer 188 or poloxamer 407; a preservative, preferably phenol, m-cresol, benzylalcohol, propylparaben, benzylalkonium chloride or benzethonium chloride.

The aqueous compositions of the present invention cover a wide range of osmolarity, including hypotonic, isotonic and hypertonic compositions. Preferably, the solutions of the invention are substantially isotonic. Preferred solutions have an osmolarity in the range of about 200 to about 500 mOsm/L. Preferably, the osmolarity is in the range of about 250 to about 350 mOsm/L. More preferably, the osmolarity is about 300 mOsm/L. In one embodiment, the solution is intended for administration to a subject by intramuscular or subcutaneous injection, and the osmolarity of the solution is selected to minimize pain upon injection.

The aqueous compositions of the present invention cover a wide range of ionic strength. The stabilizing amount of a polyethyleneimine used in the compositions of the present invention represents a contribution to ionic strength; the remaining components of the composition can be either charged (and contribute further to the ionic strength) or uncharged. In the compositions according to the present invention, the polyethyleneimine can be used to maximize the ratio of ionic strength to osmolarity. This is of particular importance in compositions of proteins that require high ionic strength, for example, in order to improve their stability, whilst at the same time they must remain isotonic or adjusted to a particular osmolarity.

In one embodiment, the invention provides an aqueous solution comprising a protein at a concentration of at least about 10 mg/mL and a polyethyleneimine wherein the ratio of ionic strength to osmolarity, both parameters originating from all non-protein excipients in the composition, is at least 3, preferably at least 5, most preferably at least 7.

The term "ionic strength", as used herein, is the following function of the concentration of all ions in a solution:

$$I = 0.5 \sum_{X=1}^{n} c_x z_x^2$$

where $c_x$ is the molar concentration of ion x (mol/L), and $z_x$ is the absolute value of the charge of ion $c_x$. The sum covers all ions (n) present in the solution. The term "osmolarity" is defined as:

$$\sum_{X=1}^{n} \varphi_x d_x c_x$$

where $c_x$ is molar concentration of component x (mol/L), $d_x$ is the number of species into which component x dissociates under the conditions studied, and $\varphi_x$ is a coefficient relating to osmotic characteristics of species x obtained experimentally under the conditions studied. Whilst in some cases, the $\varphi_x$ is higher or lower than one, it is assumed for the purpose of defining the present invention that $\varphi_x = 1$ for all formulation additives.

The term "high molecular weight species" as used herein, refers to any component of the protein content which has an apparent molecular weight at least about double the molecular weight of the parent active protein. That is, high molecular weight species are multimeric aggregates of the parent protein. The multimeric aggregates may comprise the parent protein molecules with considerably altered conformation or they may be an assembly of the parent protein units in the native or near-native conformation. The determination of high molecular weight species can be done using methods known in the art, including size exclusion chromatography, electrophoresis, analytical ultracentrifugation/sedimentation velocity, light scattering, dynamic light scattering, static light scattering and field flow fractionation.

Preferably, the composition of the invention comprises no more than 5% (by weight of total protein) high molecular weight species after storage at 40°C for at least one, two or three months. In one embodiment, the amount of high molecular weight species increases by no more than 5% (by weight of total protein), preferably no more than 3%, after storage at 40°C for at least one, two or three months. Quantitation of high molecular weight species is as percent by weight of the total protein in the composition.

In preferred embodiments, a composition of the present invention will exhibit an increase in high molecular weight species during storage which is at least 10% lower, preferably at least 25% lower, than a composition lacking polyethyleneimine but otherwise identical, following storage under the same conditions and length of time.

In an embodiment, the compositions of the invention are pharmaceutical compositions suitable for administration of a therapeutic antibody protein to a subject in need thereof. Such compositions can be used in a method for administering the therapeutic protein to the subject. The method comprises the step of administering to the subject an aqueous solution comprising the therapeutic antibody protein at a concentration of least about 10 mg/mL and a stabilizing amount of polyethyleneimine. Preferably the composition is administered by injection, such as subcutaneous, intravenous or intramuscular injection. In preferred embodiments, the concentration of the protein is sufficiently high that the total volume of each administration does not exceed about 2 mL. Preferably, the total volume of each administration does not exceed about 1.5 mL or about 1.0 mL. In one embodiment, the volume of solution of each administration is from about 0.5 to about 2 mL, preferably from about 0.5 to about 1.5 mL.

In another embodiment, the invention provides a packaged pharmaceutical composition suitable for administration to a subject in need thereof. The pharmaceutical composition comprises an aqueous solution comprising a therapeutic antibody protein at a concentration of at least about 10 mg/mL and a stabilizing amount of polyethyleneimine, wherein the weight ratio of antibody protein to polyethyleneimine is at least 20. Preferably, the volume of the solution is about 2 mL or less. In one embodiment, the volume of the solution provides an administration volume of about 0.5 to about 2 mL with sufficient overage to accommodate limitations of solution uptake via syringe. In one embodiment, the overage is from about 10% to about 20% of the administration volume. The pharmaceutical composition is preferably packaged in a vial suitable for introduction of a needle for removal of the solution. In one embodiment, the pharmaceutical composition is packaged in a glass vial with a rubber stopper. The packaged pharmaceutical composition can be provided as a kit, further comprising instructions for use and, optionally, a syringe suitable for intramuscular or subcutaneous administration.

Alternatively, the packaged pharmaceutical composition can be provided in the form of a pre-filled disposable syringe suitable for intramuscular or subcutaneous administration.

EXEMPLIFICATION

Methods

Aggregation in the aqueous protein compositions was assessed by:

(a) visual assessment (i.e. signs of visible aggregate formation); and
(b) the amount of high molecular weight species as measured by size exclusion chromatography (SEC).

SEC was also used in Example 3(B) to measure the amount of low molecular weight species.

In Example 4(B), the shear viscosity of the antibody samples was measured using an AR-G2 rotational rheometer, manufactured by TA Instruments (New Castle, Delaware, U.S.A.), using a cone and plate geometry in steady shear mode with a 40 mm diameter upper conical plate having a 1° cone angle. Testing was carried out at 20 °C. A vapour trap was used to prevent loss of volatiles from the test region. Steady shear rotation speeds were selected to obtain shear rates in the range from 10 s$^{-1}$ to 1000 s$^{-1}$. The calibration and operation of the instrument was checked using Newtonian reference fluids (ZMK GmbH, Bitterfeld-Wolfen, Germany).

Results

Aggregation was measured in aqueous compositions of antibodies following storage at indicated temperatures for an indicated period of time and compared with the level of aggregates prior to the storage (To). The antibodies were prepared in a specific background solution (also indicated) and the additional effect of a polyethyleneimine (PEI) was studied. Results are expressed in terms of:

(a) % high molecular species (HMWS) measured by SEC; and
(b) result of the visual inspection (clear/cloudy).

Example 1: Trastuzumab

A. Trastuzumab was formulated at 100 mg/ml in the following background solution: EDTA (15 mM), methionine (5 mM), Tween 80 (0.2 mg/ml). The pH was adjusted either to 5.1 or to 5.8.). The increase in aggregates at 40°C in the presence of selected excipients is shown in Table 1. The effect of PEI (average Mn ~1,800 by GPC, average Mw ~2,000 by LS) was studied in the presence of either arginine (100 mM) or a mixture of trehalose (100 mM) and sodium sulfate (100 mM). In all background solutions the presence of PEI was found to reduce considerably the rate of formation of high molecular weight species (HMWS).

Table 1. The rate of aggregation in formulations of trastuzumab (40°C).

| Trehalose (mM) | Arginine (mM) | Sulfate* (mM) | PEI (g/l) | pH | % HMWS $T_0$ | % HMWS 40°C (8 weeks) | Visual (8 weeks) |
|---|---|---|---|---|---|---|---|
| 100 | | 50 | | 5.1 | -1.5 | 5.3 | Clear |
| 100 | | 50 | | 5.8 | -1.5 | 6.8 | Clear |
| 100 | | 50 | 1 | 5.1 | -1.5 | 2.7 | Clear |
| 100 | | 50 | 1 | 5.8 | -1.5 | 3.2 | Clear |
| 100 | | 50 | 5 | 5.1 | -1.5 | 2.2 | Clear |
| 100 | | 50 | 5 | 5.8 | -1.5 | 2.2 | Clear |
| | 100 | | | 5.1 | -1.5 | 11.5 | Clear |
| | 100 | | | 5.8 | -1.5 | 8.1 | Clear |
| | 100 | | 1 | 5.1 | -1.5 | 3.2 | Clear |
| | 100 | | 1 | 5.8 | -1.5 | 2.2 | Clear |
| | 100 | | 5 | 5.1 | -1.5 | 2.2 | Clear |
| | 100 | | 5 | 5.8 | -1.5 | 2.1 | Clear |
| *as sodium sulphate | | | | | | | |

B. Trastuzumab was formulated at 100 mg/ml in the following background solution: EDTA (15 mM), Methionine (1 mM), Tween 80 (0.2 mg/ml). All formulations were adjusted to pH 5.0. The increase in aggregation at 40°C in the presence of selected excipients is shown in Table 2. The effect of a range of PEI materials ((i) PEI average Mn -600 by GPC, average Mw -800 by LS (ii) Branched PEI (BPEI) average Mn ~1,200, average Mw ~1300 by light scattering, (iii) BPEI average Mn ~1,800, average Mw -2000, (iv) Linear PEI (LPEI) average Mw 2,500, (v) LPEI average Mw 4,000, (vi) BPEI average Mw 10,000, (vii) LPEI average Mw 25,000, (viii) BPEI average Mw50-100,000, (ix) LPEI average Mn 60,000, average Mw 750,000) was studied in the presence of either arginine (80 mM) or trehalose (200 mM). In all background solutions the presence of PEI was consistently found to reduce the rate of formation of HMWS.
The results are set forth in Table 2 below.

Table 2. The rate of aggregation in formulations of Trastuzumab (40°C)

| Arginine (mM) | Trehalose (mM) | PEI (Type) conc. (mg/ml) | pH | %HMWS $T_0$ | %HMWS 40°C (4 weeks) | %HMWS 40°C (12 weeks) | Visual (12 weeks) |
|---|---|---|---|---|---|---|---|
| 80 | | | 5 | 0.64 | 0.93 | 1.38 | Clear |
| 80 | | (i)1 | 5 | 0.66 | 0.78 | 0.89 | Clear |
| 80 | | (i) 5 | 5 | 0.56 | 0.79 | 0.83 | Clear |
| 80 | | (ii)1 | 5 | 0.65 | 0.93 | 0.92 | Clear |
| 80 | | (ii) 5 | 5 | 0.64 | 0.84 | 0.85 | Clear |
| 80 | | (iii)1 | 5 | 0.55 | 0.95 | 1.14 | Clear |
| 80 | | (iii)5 | 5 | 0.68 | 0.87 | 0.77 | Clear |
| 80 | | (iv)1 | 5 | 0.58 | 0.89 | 0.88 | Clear |
| 80 | | (iv)5 | 5 | 0.59 | 0.82 | 0.80 | Clear |

(continued)

| Arginine (mM) | Trehalose (mM) | PEI (Type) conc. (mg/ml) | pH | %HMWS $T_0$ | %HMWS 40°C (4 weeks) | %HMWS 40°C (12 weeks) | Visual (12 weeks) |
|---|---|---|---|---|---|---|---|
| 80 | | (v)1 | 5 | 0.71 | 0.95 | 0.95 | Clear |
| 80 | | (vi)1 | 5 | 0.73 | 0.85 | 0.94 | Clear |
| 80 | | (vi)5 | 5 | 0.53 | 0.80 | 0.90 | Clear |
| 80 | | (vii)1 | 5 | 0.73 | 0.80 | 0.83 | Clear |
| 80 | | (vii)5 | 5 | 0.71 | 0.78 | 0.78 | Clear |
| 80 | | (viii)1 | 5 | 0.49 | 0.94 | 0.87 | Clear |
| 80 | | (viii)5 | 5 | 0.54 | 0.87 | 0.77 | Clear |
| 80 | | (ix)1 | 5 | 0.60 | 1.00 | 0.95 | Clear |
| 80 | | (ix)5 | 5 | 0.57 | 0.90 | 0.78 | Clear |
| | 200 | | 5 | 0.55 | 1.57 | 2.13 | Clear |
| | 200 | (i)1 | 5 | 0.49 | 0.90 | 0.89 | Clear |
| | 200 | (i)5 | 5 | 0.64 | 0.79 | 0.65 | Clear |
| | 200 | (ii)1 | 5 | 0.72 | 1.10 | 1.35 | Clear |
| | 200 | (ii)5 | 5 | 0.44 | 0.73 | 0.68 | Clear |
| | 200 | (iii)1 | 5 | 0.65 | 0.98 | 1.06 | Clear |
| | 200 | (iii)5 | 5 | 0.69 | 0.89 | 0.88 | Clear |
| | 200 | (iv)1 | 5 | 0.58 | 0.99 | 0.93 | Clear |
| | 200 | (iv)5 | 5 | 0.60 | 0.84 | 0.82 | Clear |
| | 200 | (v)5 | 5 | 0.65 | 0.81 | 0.67 | Clear |
| | 200 | (vi)1 | 5 | 0.59 | 1.07 | 1.27 | Clear |
| | 200 | (vi)5 | 5 | 0.57 | 0.72 | 0.82 | Clear |
| | 200 | (vii)1 | 5 | 0.68 | 0.90 | 0.86 | Clear |
| | 200 | (vii)5 | 5 | 0.69 | 0.84 | 0.68 | Clear |
| | 200 | (viii)1 | 5 | 0.69 | 1.10 | 0.96 | Clear |
| | 200 | (viii)5 | 5 | 0.52 | 1.03 | 0.92 | Clear |
| | 200 | (ix)1 | 5 | 0.63 | 1.08 | 0.89 | Clear |
| | 200 | (ix)5 | 5 | 0.58 | 0.91 | 0.71 | Clear |

C. Trastuzumab was formulated at 100 mg/ml in the following backgrounds solutions: Histidine (15 mM), Methionine (1 mM), Tween 80 (0.2 mg/ml). The pH was adjusted to 4.5, 5.5 or 6.5. The increase in aggregations at 40°C in presence of selected excipients is shown in Table 3. The effect of PEI (BPEI, average Mn ~1,800, average Mw -2000) was studied in the presence of either arginine (80 mM) or trehalose (200 mM). In all background solutions the presence of PEI was found to reduce considerably the rate of formation of HMWS.
The results are set forth in Table 3 below.

Table 3. The rate of aggregation in formulations of Trastuzumab (40°C)

| Arginine (mM) | Trehalose (mM) | PEI (mg/ml) | Sodium Benzoate | pH | %HMWS $T_0$ | %HMWS 40°C (4 weeks) | %HMWS 40°C (12 weeks) | Visual (12 weeks) |
|---|---|---|---|---|---|---|---|---|
| 80 | | | | 5 | 0.64 | 0.93 | 1.38 | Clear |
| 80 | | 2.5 | | 4.5 | 0.53 | 0.85 | 0.78 | Clear |
| 80 | | 2.5 | | 5.5 | 0.53 | 0.88 | 0.78 | Clear |
| 80 | | 2.5 | | 6.5 | 0.47 | 0.86 | 0.82 | Clear |
| 80 | | 2.5 | 10 | 5.5 | 0.51 | 0.91 | 0.89 | Clear |
| 80 | | 2.5 | 10 | 6.5 | 0.46 | 0.72 | 0.88 | Clear |
| | 200 | | | 5 | 0.55 | 1.57 | 2.13 | Clear |
| | 200 | 2.5 | | 4.5 | 0.54 | 0.71 | 0.64 | Clear |
| | 200 | 2.5 | | 5.5 | 0.47 | 0.74 | 0.59 | Clear |
| | 200 | 2.5 | | 6.5 | 0.67 | 1.06 | 1.20 | Clear |
| | 200 | 2.5 | 10 | 5.5 | 0.49 | 0.86 | 0.78 | Clear |
| | 200 | 2.5 | 10 | 6.5 | 0.45 | 0.77 | 0.80 | Clear |

Example 2: Rituximab

A. Rituximab was formulated at 100 mg/ml in the indicated formulations. All formulations were adjusted to pH 6.5 and contained 220 mM trehalose. The increase in aggregates in the presence of selected additional excipients is shown in Table 4 (40°C) and Table 5 (5°C). The effect of PEI (average Mn ~1,800 by GPC, average Mw ~2,000 by LS) was studied in the presence of either citrate (15 mM), histidine (15 mM) or a mixture of TRIS (15 mM) and sodium benzoate (15 mM). In all buffer backgrounds the presence of PEI was found to reduce considerably the rate of formation of HMWS. In addition, at 40°C, precipitation or cloudiness was observed after 12 weeks in the control formulations not containing PEI.
The results are set forth in Tables 4 and 5 below.

Table 4. The rate of aggregation in formulations of rituximab at 40°C.

| Citrate (mM) | Histidine (mM) | TRIS/ Benzoate* (mM/mM) | PEI (g/l) | % HMWS $T_0$ | % HMWS 4 weeks | Visual 4 weeks | % HMWS 12 weeks | Visual 12 weeks |
|---|---|---|---|---|---|---|---|---|
| 15 | | | | 1.8 | 7.5 | Clear | 19.8 | Cloudy |
| 15 | | | 10 | 1.4 | 2.0 | Clear | 3.6 | Clear |
| | 15 | | | 1.7 | 5.7 | Clear | N/D*** | Prec.** |
| | 15 | | 10 | 1.8 | 2.1 | Clear | 3.3 | Clear |
| | | 15/15 | | 1.7 | 4.8 | Clear | N/D*** | Prec.** |
| | | 15/15 | 10 | 1.9 | 2.1 | Clear | 3.4 | Clear |

*as sodium benzoate
**Prec. = precipitated
***N/D = Not determined

Table 5. The rate of aggregation in formulations of rituximab at 5°C.

| Citrate (mM) | Histidine (mM) | TRIS/ Benzoate* (mM/mM) | PEI (g/l) | % HMWS $T_0$ | % HMWS 4 weeks | Visual 4 weeks | % HMWS 12 weeks | Visual 12 weeks |
|---|---|---|---|---|---|---|---|---|
| 15 | | | | 1.8 | 3.1 | Clear | 4 | Clear |
| 15 | | | 10 | 1.4 | 1.8 | Clear | 2.2 | Clear |
| | 15 | | | 1.7 | 3.2 | Clear | 3.7 | Clear |
| | 15 | | 10 | 1.8 | 1.9 | Clear | 2.1 | Clear |
| | | 15/15 | | 1.7 | 2.0 | Clear | 2.9 | Clear |
| | | 15/15 | 10 | 1.9 | 2.1 | Clear | 2.3 | Clear |
| *as sodium benzoate | | | | | | | | |

B. Rituximab was formulated at 100 mg/ml in the following background solution: EDTA (0.2 mM), Methionine (1 mM), Histidine (10 mM). The pH was adjusted to 4.5, 5.5 or 6.5. The increase in aggregation at 40°C in the presence of selected excipients is shown in Tables 6 and 7. The effect of a range of PEIs ((i) PEI average Mn -600 by GPC, average Mw ~800 by LS (ii) BPEI average Mn ~1,200, average Mw ~1300 by LS, (iii) BPEI average Mw 10,000, (iv) BPEI average Mw 50-100,000) was studied in the presence of either arginine (80 mM) or trehalose (200 mM). In all background solutions the presence of PEIs was found to reduce considerably the rate of formation of HMWS. In the presence of trehalose, all formulations tested remained clear for up to 12 weeks at 40°C. In contrast, in the presence of arginine the control formulation (i.e. in the absence of PEI) showed signs of visible precipitation after 12 weeks, whilst the formulations containing PEI remained clear. The HMWS observed in the arginine-based control formulation at 8 weeks appears to be relatively low (2%, Table 6), but this is likely to be due to the fact that some of the aggregated species formed insoluble aggregates.

The results are set forth in Tables 6 and 7 below.

Table 6. The rate of aggregation in formulations of Rituximab (40°C)

| Arginine (mM) | Trehalose (mM) | PEI(Type) conc.(mg/ml) | pH | %HMWS $T_0$ | %HMWS 40°C (4 weeks) | %HMWS 40°C (8 weeks) | %HMWS 40°C (12 weeks) | Visual (12 weeks) |
|---|---|---|---|---|---|---|---|---|
| 80 | | | 6.5 | 2.2 | 3.2 | 2.0 | N/A | Cloudy |
| 80 | | (i)1 | 6.5 | 2.3 | 2.7 | 2.5 | 3.1 | Clear |
| 80 | | (ii)0.2 | 4.5 | 2.6 | 3.0 | 2.8 | 3.6 | Clear |
| 80 | | (ii)1 | 4.5 | 2.5 | 3.2 | 3.7 | 5.3 | Clear |
| 80 | | (ii)5 | 4.5 | 2.6 | 2.6 | 2.3 | 2.5 | Clear |
| 80 | | (ii)0.2 | 5.5 | 2.7 | 3.0 | 2.5 | 3.2 | Clear |
| 80 | | (ii)1 | 5.5 | 2.6 | 2.8 | 2.5 | 3.3 | Clear |
| 80 | | (ii)0.2 | 6.5 | 2.6 | 3.1 | 3.2 | 4.8 | Clear |
| 80 | | (ii)1 | 6.5 | 2.6 | 2.9 | 3.5 | 5.3 | Clear |
| 80 | | (iv)1 | 6.5 | 2.5 | 3.0 | 2.6 | 3.5 | Clear |
| | 200 | | 6.5 | 2.0 | 4.2 | 4.3 | 7.3 | Clear |
| | 200 | (i)1 | 6.5 | 2.0 | 2.9 | 2.5 | 3.1 | Clear |
| | 200 | (i)5 | 6.5 | 2.1 | 2.4 | 2.3 | 2.2 | Clear |
| | 200 | (ii)0.2 | 4.5 | 2.1 | 2.9 | 2.6 | 3.6 | Clear |
| | 200 | (ii)1 | 4.5 | 2.0 | 2.6 | 2.2 | 2.7 | Clear |

(continued)

| Arginine (mM) | Trehalose (mM) | PEI(Type) conc.(mg/ml) | pH | %HMWS $T_0$ | %HMWS 40°C (4 weeks) | %HMWS 40°C (8 weeks) | %HMWS 40°C (12 weeks) | Visual (12 weeks) |
|---|---|---|---|---|---|---|---|---|
| | 200 | (ii)5 | 4.5 | 2.1 | 2.4 | 2.0 | 2.4 | Clear |
| | 200 | (ii)0.2 | 5.5 | 2.1 | 3.1 | 2.7 | 3.8 | Clear |
| | 200 | (ii)1 | 5.5 | 2.1 | 2.7 | 3.6 | 5.8 | Clear |
| | 200 | (ii)0.2 | 6.5 | 2.1 | 3.1 | 3.1 | 4.9 | Clear |
| | 200 | (ii)1 | 6.5 | 2.0 | 2.8 | 4.5 | 7.4 | Clear |
| | 200 | (ii)5 | 6.5 | 2.1 | 2.4 | 1.9 | 2.4 | Clear |
| | 200 | (iii)5 | 6.5 | 2.2 | 2.6 | 3.2 | 4.3 | Clear |
| | 200 | (iv)5 | 6.5 | 2.1 | 2.6 | 2.2 | 4.3 | Clear |

Table 7. The rate of aggregation in formulations of Rituximab (40°C) - long term storage.

| Trehalose (mM) | (ii) PEI (mg/ml) | (iii) PEI (mg/ml) | pH | %HMWS T0 | %HMWS 40°C (4 weeks) | %HMWS 40°C (8 weeks) | %HMWS 40°C (12 weeks) | %HMWS 40°C (6 months) | Visual (6 months) |
|---|---|---|---|---|---|---|---|---|---|
| 200 | | | 6.5 | 2.0 | 4.2 | 4.3 | 7.3 | N/A | Cloudy |
| 200 | 1 | | 4.5 | 2.0 | 2.6 | 2.2 | 2.7 | 3.0 | Clear |
| 200 | 5 | | 4.5 | 2.1 | 2.4 | 2.0 | 2.4 | 1.9 | Clear |
| 200 | 5 | | 6.5 | 2.1 | 2.4 | 1.9 | 2.4 | 1.6 | Clear |
| 200 | | 5 | 6.5 | 2.2 | 2.6 | 3.2 | 4.3 | 6.5 | Clear |

Example 3: Etanercept

A. Etanercept was formulated at 50 mg/ml in the following background solution: Histidine (5 mM), Methionine (5 mM), EDTA (0.25 mM). The pH was adjusted to 5.0, 6.0 or 7.0. The increase in HMWS at 40°C in the presence of selected excipients is shown in Table 8. The effect of PEI (average Mn ~1,200, average Mw ~1300 by light scattering) was studied in the presence of either sodium lactate (100 mM) or 1,2-propanediol (250 mM) or a mixture of 1,2-propanediol (250 mM) and potassium benzoate (20 mM). In all background solutions the presence of PEI was found to reduce considerably the rate of formation of HMWS.

The results are set forth in Table 8 below.

Table 8. The rate of aggregation in formulations of Etanercept (40°C)

| *Lactate (mM) | 1,2-propanediol (mM) | PEI (mg/ml) | Potassium Benzoate (mM) | pH | % HMWS $T_0$ | %HMWS 40°C 4 weeks | %HMWS 40°C 8 weeks | %HMWS 40°C 12 weeks | Visual 12 weeks |
|---|---|---|---|---|---|---|---|---|---|
| 100 | | | | 5 | 0.8 | 7.8 | 14.4 | 17.5 | Clear |
| 100 | | 2.5 | | 5 | 1.2 | 4.3 | 6.7 | 6.4 | Clear |
| 100 | | | | 6 | 1.1 | 8.6 | 15.8 | 20.5 | Clear |
| 100 | | 2.5 | | 6 | 1.3 | 5.3 | 7.9 | 8.9 | Clear |
| 100 | | | | 7 | 1.3 | 12.1 | 24.6 | 35.7 | Clear |
| 100 | | 2.5 | | 7 | 1.2 | 6.1 | 9.4 | 10.2 | Clear |
| | 250 | | | 5 | 1.6 | 23.2 | 52.0 | 76.8 | Clear |
| | 250 | 2.5 | | 5 | 1.2 | 4.8 | 8.1 | 8.5 | Clear |
| | 250 | | | 6 | 1.6 | 22.0 | 53.7 | 82.5 | Clear |
| | 250 | 2.5 | | 6 | 1.4 | 5.6 | 10.1 | 10.4 | Clear |
| | 250 | | | 7 | 1.5 | 24.0 | 61.9 | 100.6 | Clear |
| | 250 | 2.5 | | 7 | 1.0 | 6.4 | 11.5 | 11.5 | Clear |
| | 250 | | 20 | 6 | 1.2 | 12.0 | 20.4 | 35.4 | Clear |
| | 250 | 2.5 | 20 | 6 | 1.4 | 3.9 | 6.9 | 6.6 | Clear |

*as sodium lactate

B. Etanercept was formulated at 50 mg/ml in the following background solution: Histidine (5 mM), Methionine (5 mM), EDTA (0.25 mM). The pH was adjusted to 5.0, 6.0 or 7.0. The increase in aggregation at 40°C in the presence of selected excipients is shown in Table 9. The effect of PEI (average $M_n$ ~1,200, average $M_w$ ~1300 by light scattering) was studied in the presence of either sodium lactate (100 mM) or 1,2-propanediol (250 mM) or a mixture of 1,2-propanediol (250 mM) and potassium benzoate (20 mM). In all background solutions the presence of PEI was found to reduce considerably the rate of formation of low molecular weight species (LMWS), particularly at pH 5.

The results are set forth in Table 9 below.

Table 9. The rate of low molecular weight species formation in formulations of Etanercept (40°C)

| Lactate (mM) | 1,2-propanediol (mM) | PEI (mg/ml) | Potassium Benzoate (mM) | pH | %LMWS $T_0$ | %LMWS 40°C (4 weeks) | %LMWS 40°C (8 weeks) | %LMWS 40°C (12 weeks) | Visual(12 weeks) |
|---|---|---|---|---|---|---|---|---|---|
| 100 | | | | 5 | 12.9 | 27.6 | 43.5 | 72.5 | Clear |
| 100 | | 2.5 | | 5 | 14.7 | 15.7 | 24.2 | 41.0 | Clear |
| 100 | | | | 6 | 12.6 | 17.7 | 25.2 | 43.3 | Clear |
| 100 | | 2.5 | | 6 | 15.0 | 15.3 | 20.8 | 35.8 | Clear |
| 100 | | | | 7 | 12.5 | 17.3 | 23.1 | 41.1 | Clear |
| 100 | | 2.5 | | 7 | 15.0 | 17.1 | 23.9 | 39.4 | Clear |
| 100 | | | 20 | 6 | 14.5 | 16.7 | 24.5 | 41.4 | Clear |
| 100 | | 2.5 | 20 | 6 | 15.9 | 14.3 | 20.3 | 36.9 | Clear |
| | 250 | | | 5 | 14.0 | 24.3 | 42.2 | 72.7 | Clear |
| | 250 | 2.5 | | 5 | 14.4 | 13.2 | 19.1 | 35.8 | Clear |
| | 250 | | | 6 | 15.4 | 19.0 | 31.6 | 56.9 | Clear |
| | 250 | 2.5 | | 6 | 16.2 | 13.3 | 19.0 | 36.0 | Clear |
| | 250 | | | 7 | 13.7 | 17.0 | 27.3 | 50.9 | Clear |
| | 250 | 2.5 | | 7 | 12.7 | 15.3 | 21.4 | 41.3 | Clear |
| | 250 | | 20 | 6 | 15.9 | 18.0 | 4.0 | 45.2 | Clear |
| | 250 | 2.5 | 20 | 6 | 16.2 | 14.1 | 19.0 | 38.7 | Clear |

Example 4: Fresolimumab

A. Fresolimumab was formulated at 100 mg/ml in the following background solution: Histidine (10 mM) and Tween 80 (150 mg/ml). The pH was adjusted to 5.0, 6.0 or 7.0. The increase in HMWS at 40°C in the presence of selected excipients is shown in Table 10. The effect of PEI (BPEI average Mn ~1,800, average Mw -2000) was studied in the presence of sodium chloride (150 mM), sodium sulphate (100 mM), 1,2-propanediol (250 mM) or trehalose (250 mM). In all background solutions the presence of PEI was found to reduce considerably the rate of formation of HMWS. By visual assessment, all solutions remained clear after 8 weeks.

The results are set forth in Table 10 below.

Table 10. The rate of aggregation in formulations of Fresolimumab (40°C).

| Sodium Chloride (mM) | Sodium Sulphate (mM) | 1,2-propanediol (mM) | Trehalose (mM) | PEI (mg/ml) | pH | %HMWS $T_0$ | %HMWS 40°C (4 weeks) | %HMWS 40°C (12 weeks) |
|---|---|---|---|---|---|---|---|---|
| 150 | | | | | 5 | 0.47 | 2.04 | 3.83 |
| 150 | | | | 5 | 5 | 0.43 | 1.36 | 0.80 |
| 150 | | | | | 6 | 0.49 | 2.17 | 5.20 |
| 150 | | | | 5 | 6 | 0.47 | 0.75 | 0.71 |
| 150 | | | | | 7 | 0.52 | 2.05 | 6.63 |
| 150 | | | | 5 | 7 | 0.47 | 1.27 | 0.36 |
| | 100 | | | | 6 | 0.00 | 6.05 | 9.55 |
| | 100 | | | 5 | 5 | 0.46 | 1.82 | 1.92 |
| | 100 | | | 5 | 6 | 0.47 | 2.00 | 1.79 |
| | 100 | | | 5 | 7 | 0.45 | 2.48 | 1.94 |
| | | 250 | | | 5 | 0.41 | 2.51 | 3.77 |
| | | 250 | | 5 | 5 | 0.44 | 1.87 | 0.75 |
| | | 250 | | | 6 | 0.45 | 2.36 | 3.93 |
| | | 250 | | 5 | 6 | 0.00 | 1.92 | 0.61 |
| | | 250 | | | 7 | 0.53 | 3.49 | 6.53 |
| | | 250 | | 5 | 7 | 0.48 | 1.94 | 0.74 |
| | | | 250 | 5 | 5 | 0.41 | 1.21 | 0.64 |
| | | | 250 | 5 | 6 | 0.52 | 1.07 | 0.61 |
| | | | 250 | 5 | 7 | 0.51 | 1.42 | 0.62 |

B. Fresolimumab was formulated at 100 mg/ml in the following background solution: Histidine (10 mM) and Tween 80 (150 mg/ml). All formulations were adjusted to pH 6.0. The effect of PEI (BPEI average Mw ~2000 g/mol) was studied in the presence of either sodium chloride (150 mM) or 1,2-propanediol (300 mM). In both background solutions the presence of PEI was found to reduce considerably the rate of formation of HMWS. By visual assessment, all solutions remained clear after 8 weeks.

In addition, viscosity measurements shown in Table 12 for the PEI-containing formulations indicated that low and consistent viscosity was maintained during the storage period.

The results are set forth in Tables 11 and 12 below.

Table 11. The rate of aggregation in formulations of Fresolimumab (2-8°C and 40°C)

| Sodium Chloride (mM) | 1,2-propanediol (mM) | PEI (mg/ml) | %HMWS $T_0$ | %HMWS 2-8°C (4 weeks) | %HMWS 40°C (4 weeks) | %HMWS 2-8°C (8 weeks) | %HMWS 40°C (8 weeks) |
|---|---|---|---|---|---|---|---|
| 150 | | | 0.32 | 0.23 | 1.96 | 0.26 | 2.66 |
| 150 | | 5 | 0.33 | 0.12 | 0.39 | 0.18 | 0.49 |
| | 300 | | 0.38 | 0.11 | 7.23 | 0.35 | 16.02 |
| | 300 | 5 | 0.33 | 0.24 | 0.41 | 0.18 | 0.62 |

Table 12. The viscosity of formulations of Fresolimumab (2-8°C and 40°C).

| Sodium Chloride (mM) | 1,2-propanediol (mM) | PEI (mg/ml) | Viscosity [mPa·s] $T_0$ | Viscosity [mPa·s] 2-8°C (4 weeks) | Viscosity [mPa·s] 40°C (4 weeks) | Viscosity [mPa·s] 2-8°C (8 weeks) | Viscosity [mPa·s] 40°C (8 weeks) |
|---|---|---|---|---|---|---|---|
| 150 | | 5 | 2.93 | 3.02 | 2.98 | 2.98 | 2.81 |
| | 300 | 5 | 4.74 | 4.98 | 4.75 | 5.01 | 4.52 |

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

While this invention has been particularly shown and described with references to preferred embodiments thereof, it will be understood by those skilled in the art that various changes in form and details may be made therein without departing from the scope of the invention encompassed by the appended claims. It should also be understood that the embodiments described herein are not mutually exclusive and that features from the various embodiments may be combined in whole or in part in accordance with the invention.

## Claims

1. An aqueous solution for administration to a subject by subcutaneous, intravenous or intramuscular injection comprising an antibody protein at a concentration of at least 10 mg/mL and a stabilizing amount of polyethyleneimine, wherein the weight ratio of antibody protein to polyethyleneimine is at least 20.

2. The aqueous solution of claim 1 wherein the antibody protein is a therapeutic agent.

3. The aqueous solution of claim 1 or claim 2 wherein the antibody protein is an antibody, an antibody fragment, an antibody conjugated to an active moiety, a fusion protein comprising one or more antibody fragments, or a derivative of any of the aforementioned.

4. The aqueous solution of claim 3 wherein the antibody protein is a monoclonal antibody.

5. The aqueous solution of claim 4 wherein the monoclonal antibody is trastuzumab, rituximab or fresolimumab.

6. The aqueous solution of claim 1 wherein the antibody protein is a fusion protein comprising an active protein domain fused to one or more immunoglobulin Fc fragments.

7. The aqueous solution of any one of claims 1 to 6 wherein the antibody protein concentration is between 25 mg/mL and 300 mg/mL, or is greater than 50 mg/mL.

8. The aqueous solution of any one of claims 1 to 7 wherein the polyethyleneimine concentration is from 0.5 mg/mL to 5 mg/mL.

9. The aqueous solution of any one of claims 1 to 8 wherein the polyethyleneimine has a number-average molecular weight of 5000 Da or less.

10. The aqueous solution of claim 9 wherein the polyethyleneimine has a number-average molecular weight of 2500 Da or less.

11. The aqueous solution of any one of claims 1 to 10, wherein the aqueous solution is for administration to a subject by subcutaneous injection.

12. The aqueous solution of any one of claims 1 to 11, wherein the osmolarity of the aqueous solution is in the range of 200 to 500 mOsm/L.

13. A method of reducing the rate of aggregation of an antibody protein in an aqueous solution for administration to a subject by subcutaneous, intravenous or intramuscular injection, wherein the antibody protein concentration is at least 10 mg/mL, comprising the step of adding to the solution a stabilizing amount of polyethyleneimine, wherein the weight ratio of antibody protein to polyethyleneimine is at least 20.

14. A packaged pharmaceutical composition suitable for administration to a subject in need thereof, comprising the aqueous solution of any of claims 1 to 12.

15. The packaged pharmaceutical composition of claim 14 wherein the total volume of solution for administration is 2 mL or less.

**Patentansprüche**

1. Wässrige Lösung zur Verabreichung an einen Patienten durch subkutane, intravenöse oder intramuskuläre Injektion, umfassend ein Antikörperprotein in einer Konzentration von mindestens 10 mg/ml und eine stabilisierende Menge eines Polyethylenimins, wobei das Gewichtsverhältnis von Antikörperprotein zu Polyethylenimin mindestens 20 beträgt.

2. Wässrige Lösung nach Anspruch 1, wobei es sich bei dem Antikörperprotein um ein therapeutisches Mittel handelt.

3. Wässrige Lösung nach Anspruch 1 oder Anspruch 2, wobei es sich bei dem Antikörperprotein um einen Antikörper, ein Antikörperfragment, einen mit einer aktiven Gruppierung konjugierten Antikörper, ein ein oder mehrere Antikörperfragmente umfassendes Fusionsprotein oder ein Derivat eines der oben erwähnten handelt.

4. Wässrige Lösung nach Anspruch 3, wobei es sich bei dem Antikörperprotein um einen monoklonalen Antikörper handelt.

5. Wässrige Lösung nach Anspruch 4, wobei es sich bei dem monoklonalen Antikröper um Trastuzumab, Rituximab oder Fresolimumab handelt.

6. Wässrige Lösung nach Anspruch 1, wobei es sich bei dem Antikörperprotein um ein Fusionsprotein handelt, das eine mit einem oder mehreren Immunoglobulin Fc-Fragmenten kondensierte aktive Proteindomäne handelt.

7. Wässrige Lösung nach einem der Ansprüche 1 bis 6, wobei die Antikörperproteinkonzentration zwischen 25 mg/ml und 300 mg/ml liegt oder über 50 mg/ml liegt.

8. Wässrige Lösung nach einem der Ansprüche 1 bis 7, wobei die Polyethyleniminkonzentration 0,5 mg/ml bis 5 mg/ml beträgt.

9. Wässrige Lösung nach einem der Ansprüche 1 bis 8, wobei das Polyethylenimin ein zahlenmittleres Molekulargewicht von 5000 Da oder weniger aufweist.

10. Wässrige Lösung nach Anspruch 9, wobei das Polyethylenimin ein zahlenmittleres Molekulargewicht von 2500 Da oder weniger aufweist.

**11.** Wässrige Lösung nach einem der Ansprüche 1 bis 10, wobei die wässrige Lösung für die Verabreichung an einen Patienten durch subkutane Injektion bestimmt ist.

**12.** Wässrige Lösung nach einem der Ansprüche 1 bis 11, wobei die Osmolarität der wässrigen Lösung im Bereich von 200 bis 500 mOsm/l liegt.

**13.** Verfahren zur Verminderung der Aggregationsrate eines Antikörperproteins in einer wässrigen Lösung zur Verabreichung an einen Patienten durch subkutane, intravenöse oder intramuskuläre Injektion, wobei die Antikörperproteinkonzentration mindestens 10 mg/ml beträgt, umfassend den Schritt der Zugabe einer stabilisierenden Menge von Polyethylenimin zu der Lösung, wobei das Gewichtsverhältnis von Antikörperprotein zu Polyethylenimin mindestens 20 beträgt.

**14.** Abgepackte, für die Verabreichung an einen dessen bedürftigen Patienten geeignete pharmazeutische Zusammensetzung, umfassend die wässrige Lösung nach einem der Ansprüche 1 bis 12.

**15.** Abgepackte pharmazeutische Zusammensetzung nach Anspruch 14, wobei das Gesamtvolumen an Lösung für die Verabreichung 2 ml oder weniger beträgt.

**Revendications**

**1.** Solution aqueuse pour administration à un sujet par injection sous cutanée, intraveineuse ou intramusculaire comprenant une protéine d'anticorps à une concentration d'au moins 10 mg/ml et une quantité stabilisante de polyéthylénimine, dans laquelle le rapport en poids de la protéine d'anticorps à la polyéthylénimine est d'au moins 20.

**2.** Solution aqueuse de la revendication 1 dans laquelle la protéine d'anticorps est un agent thérapeutique.

**3.** Solution aqueuse de la revendication 1 ou la revendication 2 dans laquelle la protéine d'anticorps est un anticorps, un fragment d'anticorps, un anticorps conjugué à un fragment actif, une protéine de fusion comprenant un ou plusieurs fragments d'anticorps, ou un dérivé de l'un quelconque de ceux mentionnés ci-dessus.

**4.** Solution aqueuse de la revendication 3 dans laquelle la protéine d'anticorps est un anticorps monoclonal.

**5.** Solution aqueuse de la revendication 4 dans laquelle l'anticorps monoclonal est le trastuzumab, le rituximab ou le frésolimumab.

**6.** Solution aqueuse de la revendication 1 dans laquelle la protéine d'anticorps est une protéine de fusion comprenant un domaine de protéine actif fusionné à un ou plusieurs fragments Fc d'immunoglobuline.

**7.** Solution aqueuse de l'une quelconque des revendications 1 à 6 dans laquelle la concentration de protéine d'anticorps est comprise entre 25 mg/ml et 300 mg/ml, où est supérieure à 50 mg/ml.

**8.** Solution aqueuse de l'une quelconque des revendications 1 à 7 dans laquelle la concentration de polyéthylénimine est de 0,5 mg/ml à 5 mg/ml.

**9.** Solution aqueuse de l'une quelconque des revendications 1 à 8 dans laquelle la polyéthylénimine a un poids moléculaire moyen en nombre de 5000 Da ou moins.

**10.** Solution aqueuse de la revendication 9 dans laquelle la polyéthylénimine a un poids moléculaire moyen en nombre de 2500 Da ou moins.

**11.** Solution aqueuse de l'une quelconque des revendications 1 à 10, la solution aqueuse étant pour administration à un sujet par injection sous cutanée.

**12.** Solution aqueuse de l'une quelconque des revendications 1 à 11, dans laquelle l'osmolarité de la solution aqueuse est dans la plage de 200 à 500 mOsm/l.

**13.** Procédé de réduction du taux d'agrégation d'une protéine anticorps dans une solution aqueuse pour administration à un sujet par injection sous cutanée, intraveineuse ou intramusculaire, dans lequel la concentration de protéine d'anticorps est d'au moins 10 mg/ml, comprenant l'étape d'ajout à la solution d'une quantité stabilisante de polyéthylénimine, le rapport en poids de la protéine d'anticorps à la polyéthylénimine étant d'au moins 20.

14. Composition pharmaceutique conditionnée adaptée pour administration à un sujet nécessitant celle-ci, comprenant la solution aqueuse de l'une quelconque des revendications 1 à 12.

15. Composition pharmaceutique conditionnée de la revendication 14 dans laquelle le volume total de solution pour administration est de 2 ml ou moins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010035001 A

- WO 2008084237 A

**Non-patent literature cited in the description**

- **BRYJAK, J. et al.** *Enzyme Microb. Technol.,* 1994, vol. 16, 616-621
- **ANDERSSON, M. M. et al.** *J. Biotech.,* 1999, vol. 72, 21-31

- **M. HASHEMI et al.** *Cancer Gene Therapy,* 2011, vol. 18, 12-19